# EUROPÄISCHE PATENTSCHRIFT

(11) **EP 3 552 644 B1**
(45) Veröffentlichungstag und Bekanntmachung des Hinweises auf die Patenterteilung: **07.07.2021**
(21) Anmeldenummer: 18209877.2
(22) Anmeldetag: 03.12.2018
(51) Int. Cl.: A61M 15/00, A61M 15/08, B65D 83/14, A61M 11/00, B65D 83/20, B65D 83/22, B05B 1/12, B05B 1/34, B05B 1/00, B65D 83/30, B65D 83/46

(54) **AUSTRAGKOPF FÜR DIE NASALE APPLIKATION VON FLÜSSIGKEIT AUS EINEM DRUCKSPEICHER**
APPLICATOR HEAD FOR NASAL APPLICATION OF FLUID FROM A PRESSURE ACCUMULATOR
TÊTE DISTRIBUTRICE POUR L'APPLICATION NASALE DE LIQUIDE PROVENANT D'UN ACCUMULATEUR DE PRESSION

(30) Priorität: 13.04.2018 WO PCT/EP2018/059583
(43) Veröffentlichungstag der Anmeldung: 16.10.2019
(73) Patentinhaber: Aptar Radolfzell GmbH, 78315 Radolfzell (DE)
(72) Erfinder: GREINER-PERTH, Jürgen, 78244 Gottmadingen (DE); SCHMID, Felix, 78337 Öhningen (DE)
(74) Vertreter: Patentanwaltskanzlei Cartagena

(56) Entgegenhaltungen:
- EP-A1- 0 040 851
- EP-A2- 0 729 792
- WO-A1-2009/025697
- US-A- 3 961 756
- US-A- 4 706 888
- US-A1- 2016 325 916

## Beschreibung

### ANWENDUNGSGEBIET UND STAND DER TECHNIK

Die Erfindung betrifft einen Austragkopf für die Applikation pharmazeutischer Flüssigkeiten, insbesondere für die nasale Applikation von pharmazeutischen Flüssigkeit aus einem Druckspeicher, welcher über ein Auslassventil mit einem Ventilstutzen verfügt, der gegen eine Federkraft zum Öffnen des Auslassventils kraftbeaufschlagbar ist. Die Erfindung betrifft weiterhin auch einen Spender mit einem solchen Austragkopf.

Ähnliche Austragköpfe und Spender sind beispielsweise aus der DE 102012200545 A1 bekannt. Die in dieser Schrift offenbarten Austragköpfe verfügen über ein Außengehäuse, welches einerseits die Basis zur Anbindung an einem Druckspeicher beinhaltet und andererseits die Außenschale eines Nasalapplikators mitsamt Austragöffnung bildet. In den länglichen Nasalapplikator ist ein Innenbauteil eingeschoben, welches gemeinsam mit der Außenschale der Fluidführung von Flüssigkeit zur Austragöffnung dient.

Auch aus dem US Design Patent mit der Publikationsnummer US D 659,531 S ist eine ähnliche Gestaltung bekannt.

Weitere Austragköpfe und Spender sind aus folgenden Schriften bekannt: US 3,961,756, EP0040851, EP 0729792, US 2016/0325916, US 4,706,888.

Die genannten Austragköpfe werden für den Vertrieb von Medikamenten mit vergleichsweise geringem Verkaufspreis verwendet. Es ist daher erforderlich, dass die Herstellung der Austragköpfe preisgünstig möglich ist. Die diesbezügliche vorteilhafte einstückige Gestaltung des Außengehäuses mit Basis und Nasalapplikator ist zur Erzielung geringer Kosten gut geeignet. Allerdings sind auch Nachteile gegeben. Das Zuführen des flüssigen Kunststoffes im Rahmen des Spritzguss-Vorgangs erfolgt üblicherweise im Bereich der Basis. Die Austragöffnung ist daher vergleichsweise weit vom Zuführpunkt entfernt, was zu einer mitunter nicht zufriedenstellenden Fertigungsgenauigkeit an der Austragöffnung und an Fluidleitflächen stromaufwärts der Austragöffnung führt. Dies ist von Nachteil, da hierdurch das Austragverhalten der Flüssigkeit von Spender zu Spender variieren kann und nicht mehr dem beabsichtigten Austragverhalten entspricht.

Aufgabe der Erfindung ist es daher, einen gattungsgemäßen Spender dahingehend weiterzubilden, dass eine hohe Fertigungsgenauigkeit im Bereich der Austragöffnung erzielt wird, ohne die Komplexität der Fertigung relevant zu erhöhen.

### AUFGABE UND LÖSUNG

Die Erfindung gemäß Anspruch 1 betrifft einen Austragkopf für die nasale Applikation von pharmazeutischen Flüssigkeiten aus einem Druckspeicher, welcher über ein Auslassventil mit einem Ventilstutzen verfügt, der gegen eine Federkraft zum Öffnen des Auslassventils kraftbeaufschlagbar ist.

Ein erfindungsgemäßer Austragkopf verfügt über einen Applikator, in Form eines Nasalapplikators, an dessen Ende eine Austragöffnung vorgesehen ist, die durch einen Applikatorkanal des Nasalapplikators hindurch mit einem Einlass des Applikators und damit mittelbar mit dem Flüssigkeitsspeicher verbunden ist. Unter der Austragöffnung im Sinne der Erfindung ist das Ende des Applikatorkanals zu verstehen, also der Übergangspunkt, an dem die druckbeaufschlagte Flüssigkeit in eine äußere Umgebung unter Umgebungsdruck abgegeben wird und dort je nach Art des Austritts durch die Austragöffnung als freier Jet oder als Sprühstrahl vorliegt. Die Austragöffnung weist vorzugsweise eine minimale Querschnittsfläche von weniger als 4 mm² auf, insbesondere von weniger als 2 mm².

Der erfindungsgemäße Applikator verfügt weiter über ein Innenbauteil und ein Außenbauteil. Das Außenbauteil ist als vom Innenbauteil getrenntes Bauteil vorgesehen und unmittelbar oder mittelbar am Innenbauteil in einer Weise angebracht, durch die es rein rotativ gegenüber dem Innenbauteil beweglich ist. Diese rein rotative Beweglichkeit kann durch eine entsprechende axial sperrende Kopplung von Innenteil und Außenteil bewirkt sein. Sie kann jedoch auch dadurch bewirkt sein, dass das Innenbauteil zwar grundsätzlich gegenüber dem Außenbauteil axial beweglich ist, jedoch reibschlüssig an diesem gehalten ist und keinerlei Relativkraftbeaufschlagung durch den Benutzer von außen möglich ist, mittels derer Innenbauteil und Außenbauteil gegeneinander axial verlagert werden. Dies wird im Weiteren noch näher erläutert.

Die Ausbildung des drehbaren Außenbauteils als von einer Basis des Austragkopfes separates Bauteil ist von Vorteil, da hierdurch eine Austauschbarkeit des Außenbauteils erzielt werden kann, die aus Hygienegründen von Vorteil ist. Insbesondere die Austragöffnung, an der nach der Nutzung Flüssigkeit verbleiben kann, kann schnell verkeimen. Durch einen Austausch des Außenbauteils werden alle oder zumindest besonders gefährdete Bereiche ausgetauscht. Auch die Anpassung des Austragkopfes an eine bestimmte Zielgruppe ist durch das separate Außenbauteil möglich. So kann insbesondere eine erwachsenengerechte bzw. kindgerechte Gestaltung durch die Wahl des passenden Außenbauteils erzielt werden. Dabei ist sowohl möglich, dass bereits herstellerseitig eine zielgruppenspezifische Anpassung erfolgt, als auch, dass mehrere Außenbauteile an den Endkunden mitgeliefert werden, so dass er selbst die Anpassung einmalig oder fallweise vornehmen kann.

Im Falle einer Gestaltung des Applikators als Nasalapplikator wird dieser bestimmungsgemäß in ein Nasenloch des Nutzers eingeführt. Zu diesem Zweck handelt es sich um einen länglichen, schlanken Applikator, der vorzugsweise eine sich zum distalen Ende und der Austragöffnung verjüngende Formgebung aufweist. Vorzugsweise ragt der Nasalapplikator ausgehend vom Niveau einer Betätigungsfläche mindestens 20 mm hervor. Seine frei von der Basis bzw. der Betätigungsfläche hervorstehende Länge ist vorzugsweise mindestens um Faktor 2, insbesondere vorzugsweise mindestens um Faktor 2,5, größer als der maximale Durchmesser des Nasalapplikators.

Vorzugsweise verfügt der Austragkopf über eine Basis und eine daran vorgesehene Kopplungseinrichtung, mittels derer der Austragkopf am Druckspeicher befestigbar ist. Er verfügt weiterhin vorzugsweise über eine verlagerbare Betätigungsfläche, die gegenüber der genannten Basis verlagerbar ist, und an der ein als Hohlrohr ausgestalteter Stößel vorgesehen ist, der zum Zwecke der Kraftbeaufschlagung des Ventilstutzens des Druckspeichers gemeinsam mit der Betätigungsfläche verlagerbar ist. Vorzugsweise ist die Betätigungsfläche mit einer Strukturierung, beispielsweise einer Riffelung versehen. Der genannten Applikator erstreckt sich von der Betätigungsfläche aus nach außen.

Die Basis eines erfindungsgemäßen Austragkopfes ist jener Teil, der bestimmungsgemäß am Druckspeicher angekoppelt wird. Hierfür ist die Kopplungseinrichtung vorgesehen, die insbesondere vorzugsweise über angeformte Rastkanten auf dem Druckspeicher aufgeschnappt wird. Die Basis verfügt hierfür vorzugsweise über eine ringartige Struktur mit nach innen weisenden Rastnasen. Bei bevorzugt mit einem erfindungsgemäßen Austragkopf verwendeten Druckspeichern ist eine Crimp-Verbindung zwischen einem Deckel und einem Mantel vorgesehen, welche vorzugsweise zum Aufschnappen der Rastnasen verwendet wird.

Der Applikator, insbesondere in Form eines Nasalapplikators, verfügt erfindungsgemäß über ein Innenbauteil, welches vorzugsweise einstückig mit der Betätigungsfläche und/oder der Basis verbunden ist oder axial verschieblich und rotativ fixiert an einem Führungsbauteil angebracht ist, welches seinerseits einstückig mit der Betätigungsfläche und/oder der Basis verbunden ist. Weiterhin verfügt er über ein Außenbauteil, welches als vom Innenbauteil getrenntes Bauteil ausgebildet ist und das Innenbauteil umgebend an diesem angebracht ist. Die Austragöffnung ist als Durchbrechung des Außenbauteils vorgesehen. Ein zur Austragöffnung führender Applikatorkanal ist gemeinsam durch das Außenbauteil und das Innenbauteil begrenzt.

Das Außenbauteil und die Betätigungsfläche des Austragskopfes weisen vorzugsweise unterschiedliche Farbgebungen und/oder unterschiedliche Materialen auf. Die Zweifarbigkeit von Basis und Außenbauteil kann insbesondere zum Zwecke der ästhetischen Verbesserung genutzt werden, jedoch auch, um den Austragkopf an eine unternehmensspezifische typische Farbgebung anzupassen. Die Verwendung verschiedener Materialien gestattet es darüber hinaus, jeweils ideal angepasste Materialien für das Außenbauteil und die Basis mitsamt Betätigungshandhabe zu wählen. So könnte das Außenbauteil beispielsweise aus einem Material gefertigt sein, welches durch Beschichtung oder inhärente Materialeigenschaften Bakterienwachstum verhindert oder welches in Kontakt mit der Haut des Nutzers als haptisch angenehm empfunden wird, während das Hauptbauteil mit Basis und Betätigungshandhabe aus einem anderen Material bestehen kann.

Vorzugsweise ist die Betätigungsfläche schwenkbeweglich an der Basis angebracht, insbesondere vorzugsweise durch eine einstückig die Basis und die Betätigungsfläche verbindende Kunststoffbrücke, die als Schwenkgelenk agiert. Insbesondere von Vorteil ist es weiterhin, wenn auf einer der Kunststoffbrücke gegenüberliegenden Seite der Betätigungsfläche im Lieferzustand eine Sicherungsbrücke vorgesehen ist, die die Betätigungsfläche und die Basis einstückig verbindet und bei Erstbetätigung bestimmungsgemäß bricht. Die genannte einstückige Gestaltung mit verformbarer Kunststoffbrücke als Gelenk ist im Sinne eines Aufbaus aus wenigen Einzelteilen von Vorteil. Die Betätigungsfläche ist abseits der Kunststoffbrücke gegenüber der Basis durch einen Schlitz freigeschnitten, so dass sie beweglich ist. Durch die Sicherungsbrücke wird erreicht, dass es vor Inbetriebnahme nicht zu einem ungewollten Austrag von Flüssigkeit kommt, da die Inbetriebnahme zum Zwecke des Brechens dieser Sicherungsbrücke eine stärkere Kraftbeaufschlagung erfordert.

Eine durch die Austragrichtung der Austragöffnung und/oder durch eine die Rotationsmittelachse des Außenbauteils gegenüber dem Innenbauteil definierte Achse schließt vorzugsweise mit einer Aufsetzrichtung des Austragkopfs auf einen Druckspeicher einen Winkel zwischen 5° und 85° ein, vorzugsweise einen Winkel zwischen 10° und 50°. Diese Anwinkelung ist bei gattungsgemäßen Austragköpfen üblich. Sie gestattet eine vorteilhafte Verwendung, bei der der Druckspeicher, dessen Haupterstreckungsrichtung vorzugsweise mit der Aufsetzrichtung übereinstimmt, leicht angewinkelt gehalten werden kann und der Nasalapplikator dann in passendem Winkel in ein Nasenloch eingeschoben werden kann. Bei einem erfindungsgemäßen Austragkopf ist vorzugsweise weiterhin vorgesehen, dass die Betätigungsfläche und der Nasalapplikator nebeneinander angeordnet sind, so dass eine asymmetrische Formgebung des Austragkopfes gegeben ist.

Die Austragöffnung eines erfindungsgemäßen Austragkopfes ist als Durchbrechung des Außenbauteils vorgesehen und innenseitig von einer stirnseitigen Innenfläche umgeben. Der stirnseitigen Innenfläche gegenüberliegend ist eine Stirnfläche des Innenbauteils vorgesehen, welche flächig an der stirnseitigen Innenfläche anliegt.

An der stirnseitigen Innenfläche des Außenbauteils und/oder an der Stirnfläche des Innenbauteils sind erfindungsgemäß mindestens zwei Zuströmkanäle in Form von Zuströmnuten ausgebildet, die in Abhängigkeit einer Drehstellung des Außenbauteils zum Innenbauteil mit einem Zuströmbereich stromaufwärts der Zuströmnuten und somit dem Flüssigkeitsspeicher verbunden oder hiervon getrennt werden.

Die genannten Zuströmnuten verbinden somit den Zuströmbereich, in den Flüssigkeit aus dem Flüssigkeitsspeicher zunächst strömt, mit der Austragöffnung. Dabei ist es durch mindestens zwei Zuströmnuten mit unterschiedlicher Gestaltung möglich, die Austragcharakteristik der durch die Austragöffnung hindurch abgegebenen Flüssigkeit zu beeinflussen. Wird in einer ersten Drehstellung eine erste der Zuströmnuten durchströmt, während die zweite Zuströmnut verschlossen ist, so bestimmt die erste Zuströmnut und deren Formgebung, Ausrichtung und Drosselwirkung die Austragcharakteristik. Handelt es sich beispielsweise um eine radial ausgerichtete Zuströmnut, so führt dies nicht zu einer Verwirbelung und die Flüssigkeit wird als so genannter Jet ausgetragen. Wird in einer zweiten davon abweichenden Drehstellung die zweite Zuströmnut statt der ersten geöffnet, so bestimmt deren Formgebung, Ausrichtung und Drosselwirkung die Austragcharakteristik. Handelt es sich beispielsweise um eine nicht-radial ausgerichtete Nut, insbesondere eine Tangentialnut, die zur Radialrichtung angewinkelt ausgerichtet ist, so kann die Flüssigkeit einen Drall erhalten, der beim Austrag durch die Austragöffnung die Ausbildung eines Sprühstrahls begünstigt.

Grundsätzlich kann das durch Drehen des Außenbauteils bewirkte Öffnen und/oder Schließen der Zuströmnuten auch alleine dafür genutzt werden, unterschiedliche Flüssigkeitsmengen auszutragen, also quasi eine einstellbare Drosselwirkung zu erzielen. Insbesondere von Vorteil ist es jedoch, wenn Zuströmnuten zweier unterschiedlicher Arten vorgesehen sind, nämlich mindestens eine im Wesentlichen radial ausgerichtete Radialnut und mindestens eine demgegenüber bezogen auf die Austragöffnung angewinkelte Tangentialnut, deren mittlere Erstreckungsrichtung als nicht fluchtend zur Austragöffnung vorgesehen ist. Die Radialnut oder die Radialnuten dienen dabei der Erzeugung des bereits genannten Jets, während die Tangentialnut oder Tangentialnuten der Bildung eines Wirbels und damit eines Sprühstrahl dienen. Insbesondere vorzugsweise sind von den Radialnuten und/oder den Tangentialnuten jeweils mehrere vorgesehen, vorzugsweise jeweils mindestens drei, die insbesondere vorzugsweise über den Umfang gleichverteilt sind, um einen gleichmäßigen und im Wesentlichen rotationssymmetrischen Austrag zu bewirken.

Die mindestens zwei Zuströmnuten, insbesondere also die mindestens eine Radialnut und die mindestens eine Tangentialnut, sind beide im Bereich der stirnseitigen Innenfläche des Außenbauteils und der Stirnfläche des Innenbauteils vorgesehen, wobei sie vorzugsweise derart getrennt angeordnet sind, dass keine Flüssigkeit ungewollt von einer Zuströmnut in die jeweils andere Zuströmnut gelangen kann und entlangdererzur Austragöffnung strömen kann.

Um die Zuströmnuten vorteilhaft gegeneinander isolieren zu können, ist eine Gestaltung von Vorteil, bei der mindestens eine Zuströmnut in der stirnseitigen Innenfläche des Außenbauteils und mindestens eine Zuströmnut in der Stirnfläche des Innenbauteils vorgesehen ist. Es werden somit beide aneinander angepressten Flächen, die stirnseitige Innenfläche des Außenbauteils und die Stirnseite des Innenbauteils, zur Anordnung der Zuströmnuten genutzt. Hierdurch wird deren sichere Isolierung voneinander auch bei geringer Baugröße ermöglicht. Insbesondere, wenn Radial- und Tangentialnuten vorgesehen sind und vor allem wenn jeweils mehrere Radial- und Tangentialnuten vorgesehen sind, ist die Anordnung der Zuströmnuten auf die stirnseitige Innenfläche und die Stirnfläche von Vorteil.

Vorzugsweise liegen das Innenbauteil und das Außenbauteil nicht nur im Bereich der stirnseitigen Innenfläche und der Stirnfläche dicht aneinander an, sondern auch im Bereich einer an die stirnseitige Innenfläche des Außenbauteils angrenzenden zylindrischen oder im Wesentlichen zylindrischen Innenfläche und einer an die Stirnfläche des Innenbauteils angrenzenden zylindrischen oder im Wesentlichen zylindrischen Außenfläche.

Dabei ist mindestens in einer der genannten beiden zylindrischen oder im Wesentlichen zylindrischen Flächen eine Versorgungsnut vorgesehen, durch die hindurch mindestens eine Zuströmnut mit Flüssigkeit aus dem Zuströmbereich versorgbar ist. Insbesondere vorzugsweise sind in den Flächen mehrere solche Versorgungsnuten vorgesehen, die je nach Drehstellung unterschiedliche Zuströmnuten mit Flüssigkeit versorgen.

Wie bereits in Hinblick auf die Stirnfläche und die stirnseitige Innenfläche in Bezug auf die Zuströmnuten erörtert, ist vorzugsweise auch im Bereich der zylindrischen Innenfläche und der zylindrischen Außenfläche vorgesehen, dass sowohl in der Innenfläche als auch in der Außenfläche Versorgungsnuten vorgesehen sind. Es hat sich gezeigt, dass so ein besonders vorteilhafter Aufbau möglich ist, mittels dessen die Flüssigkeitszufuhr zu den unterschiedlichen Zuströmnuten zugelassen und unterbrochen werden kann. Vorteilhafterweise verlaufen alle Strömungspfade vom Zuströmbereich zur Austragöffnung jeweils partiell durch eine Nut im Innenbauteil und partiell durch eine Nut im Außenbauteil.

Es kann ein erster Flüssigkeitspfad vorgesehen sein, der im Falle der entsprechenden Drehstellung des Außenbauteils gegenüber dem Innenbauteil durch einen in der zylindrischen Außenfläche des Innenbauteils vorgesehene Versorgungsnut verläuft, wobei sich diese Versorgungsnut bis zur Stirnfläche des Innenbauteils erstreckt. Dieser Flüssigkeitspfad verläuft dann vorzugsweise weiter in eine an der stirnseitigen Innenfläche des Außenbauteils vorgesehene Zuströmnut bis zur Austragöffnung. Ist das Außenbauteil demgegenüber aber verdreht, so endet dieser Flüssigkeitspfad im Bereich der durch die Versorgungsnut durchbrochenen Stirnfläche, da er von der stirnseitigen Innenfläche des Außenbauteils blockiert wird.

In ähnlicher Weise kann ein Flüssigkeitspfad bzw. ein zweiter Flüssigkeitspfad durch eine Versorgungsnut in derzylindrischen Innenfläche des Außenbauteils verlaufen, sich jedoch dabei nicht bis auf Höhe der Stirnfläche des Innenbauteils erstrecken. Stattdessen kann dieser Flüssigkeitspfad dann bei entsprechender Drehstellung des Außenbauteils gegenüber dem Innenbauteil weiter durch eine Zuströmnut verlaufen, die sich auf der Stirnfläche des Innenbauteils bis zur angrenzenden zylindrischen Außenfläche erstreckt.

Die jeweiligen Zuströmnuten der beiden genannten Flüssigkeitspfade sind dabei derart angeordnet, dass je nach Drehstellung immer nur ein Teil der Zuströmnuten mit ihrer jeweiligen Versorgungsnut verbunden sind, während ein anderer Teil der Zuströmnuten von ihrer jeweiligen Versorgungsnut getrennt ist. Beide Flüssigkeitspfade verlaufen im geöffneten Zustand vorzugsweise sowohl durch Nuten im Innenbauteil als auch durch Nuten im Außenbauteil.

Von Vorteil ist es, wenn das Innenbauteil und das Außenbauteil in einer Drehstellung derart angeordnet sind, dass alle Zuströmkanäle zur Austragöffnung unterbrochen sind und der Austragkopf somit verschlossen ist. Bei einem Austragkopf mit zwischen zwei Endlagen limitierter Drehbeweglichkeit des Außenbauteils gegenüber dem Innenbauteil ist es von Vorteil, wenn in beiden Endlagen Flüssigkeitspfade mit unterschiedlicher Austragcharakteristik geöffnet sind und in einer Zwischenlage der Austragkopf in der genannten Art verschlossen ist.

Demgegenüber noch von Vorteil ist es, wenn im Zuströmbereich stromaufwärts der genannten Zuströmnuten und gegebenenfalls vorzugsweise auch der genannten Versorgungsnuten ein Schaltventil vorgesehen ist, welches in Abhängigkeit der Drehstellung des Außenbauteils zum Innenbauteil die Flüssigkeitszufuhr zu den Zuströmnuten unterbricht.

Mit einer solchen Gestaltung wird erreicht, dass nicht erst im Bereich der Stirnfläche und der stirnseitigen Innenfläche oder im daran angrenzenden zylindrischen Bereich der Versorgungsnuten die Flüssigkeit am Austritt durch die Austragöffnung gehindert wird, sondern bereits vor dem Einströmen in die Zuströmnuten bzw. die Versorgungsnuten an einem separaten Schaltventil. Dieses Schaltventil ist somit insbesondere vorzugsweise vor jener Stelle in den Flüssigkeitspfaden angeordnet, an dem sich die Flüssigkeitspfade für unterschiedliche Austragcharakteristiken voneinander trennen.

Das Schaltventil ist vorzugsweise durch eine Stufe am Innenbauteil und am Außenbauteil vorgesehen, also im Bereich eines Querschnittswechsels, wobei in den jeweiligen Stufen ein Ventilkanal vorgesehen ist. Nur wenn die Ventilkanäle in einer definierten Drehstellungen von Innenbauteil und Außenbauteil miteinander fluchten, kann Flüssigkeit hindurchströmen. Vorzugsweise sind an mindestens einem der beiden Bauteile, vorzugsweise an beiden Bauteilen, zwei solche Ventilkanäle vorgesehen, so dass in zwei definierten Drehstellungen Flüssigkeit zuströmen kann. Diese beiden Drehlagen können dann jene sein, die aufgrund der Gestaltung der Versorgungsnuten und Zuströmnuten unterschiedliche Austragcharakteristiken, insbesondere als Spray und als Jet, verursachen.

Um den ungewollten Wechsel von Flüssigkeit zwischen den Zuströmnuten zu unterbinden, also insbesondere einen Flüssigkeitsstrom aus einer Tangentialnut in einer Radialnut oder aus einer Radialnut in eine Tangentialnut, liegen die Stirnflächen des Innenbauteils und die stirnseitige Innenfläche des Außenbauteils dichtend aneinander an. Um trotz der Toleranzen im Fertigungsverfahren der vorzugsweise als Kunststoffteile gebildeten Bauteile, des Innenbauteils und des Außenbauteils, zu verhindern, dass die Stirnfläche und die stirnseitige Innenfläche sich voneinander abheben, besteht eine bauliche Möglichkeit darin, das Innenbauteil und das Außenbauteil mit einer Federeinrichtung zu versehen, mittels derer sie gegeneinander gepresst werden. Unter einer Federeinrichtung wird dabei eine elastisch verformte Struktur verstanden, deren Rückstellkraft die Stirnfläche und die stirnseitige Innenfläche gegeneinander presst.

Besonders vorteilhaft ist dabei eine Gestaltung, bei der das Außenbauteil und das Innenbauteil über schräggestellte Spannflächen verfügen, die durch elastische Verformung des Innenbauteils oder insbesondere des Außenbauteils in einer Radialrichtung aneinander angepresst werden und hierdurch mittelbar das Außenbauteil und das Innenbauteil aneinander anpressen. Hierbei wird ausgenutzt, dass das Außenbauteil das Innenbauteil umgibt und durch dieses elastisch radial aufgeweitet sein kann. Die entsprechende Rückstellkraft wird im Bereich der schräggestellten Spannflächen umgelenkt, so dass in der gewünschten Weise die Stirnfläche des Innenbauteils und die stirnseitige Innenfläche des Außenbauteils aneinander gepresst werden, so dass Flüssigkeit nicht von einer Zuströmnut in eine andere gelangen kann.

Von besonderem Vorteil ist es dabei, wenn das Innenbauteil und/oder das Außenbauteil eine von der Kreisform abweichende Formgebung der Spannflächen oder anderweitiger aufeinander zu weisender Kontaktflächen aufweisen, so dass bei einer Rotation des Außenbauteils gegenüber dem Innenbauteil mindestens eines der Bauteile eine variierende Verformung zeigt, die insbesondere in zwei Endlagen minimal ist und ggf. einer weitere Zwischenlage. Hierdurch ergibt sich eine besondere Stabilität der beiden Endlagen, die vorzugsweise zwei Ausbringungsarten - insbesondere als Jet und als Sprühstrahl - definieren. Eine Zwischenlage kann dem oben beschriebenen Schließzustand zugeordnet sein. Ein Verdrehen aus den Endlagen und ggf. aus der genannten Zwischenlage, geht mit einer Vergrößerung der Verformung eines der Bauteile, insbesondere des Außenbauteils, einher, so dass ein solches Verdrehen kaum unbeabsichtigt stattfinden kann.

Bei einer Variante der Erfindung ist das Innenbauteil des Applikators einstückig mit der Betätigungsfläche verbunden. Durch eine solche Ausgestaltung lässt sich ein Austragkopf erzielen, der aus nur zwei Bauteilen besteht, nämlich einem ersten Bauteil, umfassend die Basis, die Betätigungshandhabe mit Betätigungsfläche sowie das Innenbauteil des Nasalapplikators, wobei mindestens die Betätigungshandhabe durch Materialverformbarkeit gegenüber der Basis verlagerbar ist, und einem zweiten Bauteil, welches das Außenbauteil des Nasalapplikator bildet. Es ist somit eine sehr kostengünstige Bauweise möglich.

Bei einer alternativen Variante weist der Austragkopf ein Führungsbauteil auf, welches vorzugsweise einstückig mit der Betätigungsfläche und/oder der Basis ausgebildet ist. Das Innenbauteil ist dagegen bei dieser Variante nicht einstückig mit Betätigungsfläche oder Basis verbunden, sondern axial verschieblich und rotativ fixiert am Führungsbauteil angebracht. Das Außenbauteil ist ebenfalls am Führungsbauteil befestigt, allerdings drehbar.

Bei dieser alternativen Variante sind also das Außenbauteil und das Innenbauteil nicht zwingend unmittelbar aneinander axial fixiert, sondern vor allem über das Führungsbauteil, welches vorzugsweise einstückig mit der Basis ausgebildet ist. Obwohl das Innenbauteil und das Außenbauteil aufgrund der axialen Beweglichkeit des Innenbauteils gegenüber dem Führungsbauteil grundsätzlich axial gegeneinander verlagerbar sind, findet eine solche Verlagerung nicht statt. Die axiale Verlagerbarkeit dient stattdessen dem Zweck, das Innenbauteil mit besonders hoher Kraft gegen das Außenbauteil zu pressen, um Leckage im Bereich der Zuströmkanäle zu verhindern.

Hierfür ist vorzugsweise vorgesehen, dass das Innenbauteil und das Führungsbauteil gemeinsam eine Druckkammer begrenzen, wobei das Innenbauteil eine zur Druckkammer gewandte Kolbenfläche aufweist, deren Kraftbeaufschlagung durch Flüssigkeitsdruck in der Druckkammer in Richtung der Austragöffnung wirkt. Als Kolbenfläche wird die Querschnittsfläche angesehen, auf die der Flüssigkeitsdruck in Richtung der Austragöffnung wirkt. Diese Querschnittsfläche muss größer sein als eine in entgegengesetzte Richtung wirkende Querschnittsfläche im Bereich einer Abdichtung zwischen Außenbauteil und Innenbauteil.

Ein Anstieg des Flüssigkeitsdrucks in Folge einer Betätigung durch den Benutzer führt daher dazu, dass das Innenbauteil gegen das Außenbauteil gepresst wird. Dies gestattet es insbesondere, im genannten Schließzustand tatsächlich zu gewährleisten, dass keinerlei Flüssigkeit austritt, selbst wenn das Druckspeicherventil geöffnet wird. Bei einer Bauweise mit einem separaten Schaltventil im Bereich von Stufen des Innenbauteils und des Außenbauteils bewirkt diese Bauweise, dass das Schaltventil im geschlossenen Zustand vom Flüssigkeitsdruck zugepresst wird.

Ähnlich wie oben zur Verbindung von Innenbauteil und Außenbauteil beschrieben, kann auch bei einer Gestaltung mit Führungsbauteil vorgesehen sein, dass das Führungsbauteil und das Außenbauteil mit einer Federeinrichtung versehen sind. Es kann insbesondere eine Federeinrichtung vorgesehen sein, die zwischen dem Führungsbauteil und dem Außenbauteil wirkt und das Außenbauteil in Richtung der der Basis zieht und damit auf das Innenbauteil drückt.

Das Außenbauteil und das Führungsbauteil können schräggestellte Spannflächen aufweisen, die durch elastische Verformung des Führungsbauteils oder des Außenbauteils in einer Radialrichtung aneinander angepresst werden und hierdurch mittelbar das Außenbauteil und das Führungsbauteil aneinander anpressen.

Auch hier ist es von besonderem Vorteil, wenn das Führungsbauteil und/oder das Außenbauteil eine von der Kreisform abweichende Formgebung der Spannflächen oder anderer Kontaktflächen aufweisen, so dass bei einer Rotation des Außenbauteils gegenüber dem Innenbauteil mindestens eines der Bauteile eine variierende Verformung zeigt, die insbesondere in zwei Endlagen minimal ist und ggf. in einer weiteren Zwischenlage.

Auch kann auch eine Federeinrichtung vorgesehen sein, die zwischen dem Führungsbauteil und dem Innenbauteil wirkt. Eine solche Federeinrichtung zwischen dem Führungsbauteil und dem Innenbauteil kann insbesondere als metallische Druckfeder oder Druckfeder aus Kunststoff ausgebildet sein, die das Innenbauteil gegen das Außenbauteil drückt. Die Feder kann insbesondere eine Schraubenfeder sein.

Die Erfindung betrifft weiterhin auch einen Spender zum Austrag pharmazeutischer Flüssigkeiten mit einem Austragkopf der vorbeschriebenen Arten. Dieser Austragkopf ist an einem Druckspeicher des Spenders befestigt, in welchem pharmazeutische Flüssigkeit unter Druck gelagert ist und der seinerseits über ein Auslassventil verfügt. Das Auslassventil verfügt über einen Ventilstutzen, der gegen eine Federkraft zum Öffnen des Ventils mittels des Stößels des Austragkopfes kraftbeaufschlagbar ist.

Der Druckspeicher wird vorzugsweise durch eine bezüglich der Außenform rotationssymmetrische Mantelwandung begrenzt, die einstückig oder mehrstückig mit einem vorzugsweise bombierten Boden ausgebildet ist. Das integrierte Ventil ist vorzugsweise in ein Deckelteil integriert, welches mittels einer Crimp-Verbindung mit der Mantelfläche verbunden wird. Der Verbindungsbereich zwischen Mantelfläche und Deckelteil bildet vorzugsweise jene Rastkante, im Bereich derer der Austragkopf auf den Druckspeicher aufgerastet ist.

Einem Druckspeicher ist es zu eigen, dass die darin gespeicherte Flüssigkeit stets unter Druck steht, so dass der Druck nicht für einen Austragvorgang erzeugt werden muss. Für die Ausübung des Drucks sind verschiedene Möglichkeiten gegeben, so die Druckbeaufschlagung per Treibgas oder mittels komprimierter Luft im Flüssigkeitsspeicher oder insbesondere in einem Zwischenbereich zwischen einem Beutel im Druckspeicher und der Mantelwandung des Druckspeichers.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.

Fig. 1A und 1B zeigen einen erfindungsgemäßen Spender in geschnittener und ungeschnittener Gesamtdarstellung.

In den Fig. 2A bis 2I ist ein erstes Ausführungsbeispiel des Austragkopfes im Detail dargestellt.

In den Fig. 3A bis 3G ist ein zweites Ausführungsbeispiel des Austragkopfes im Detail dargestellt.

In Fig. 3H ist eine Variante des zweiten Ausführungsbeispiels des Austragkopfes dargestellt.

### DETAILLIERTE BESCHREIBUNG DERAUSFÜHRUNGSBEISPIELE

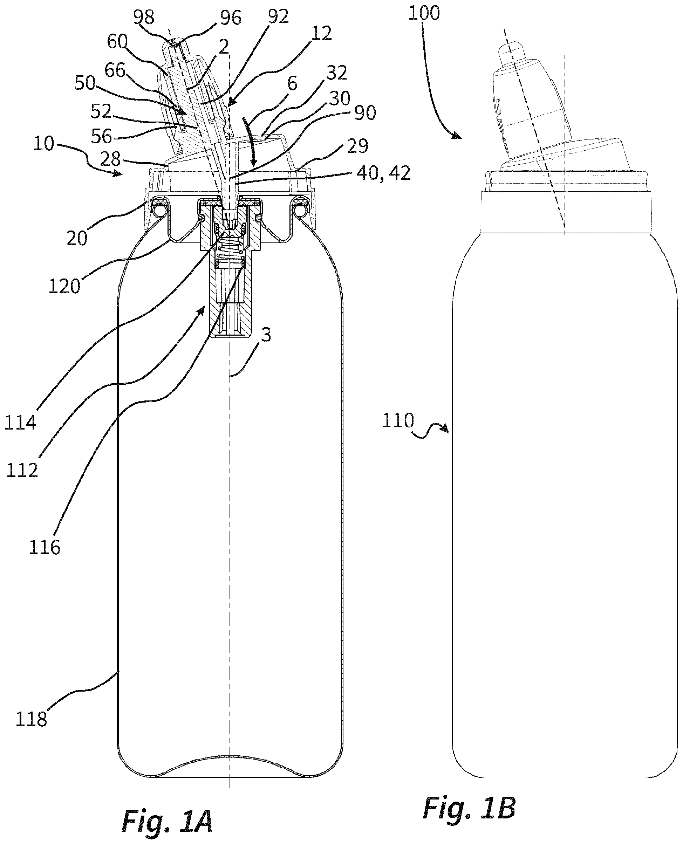

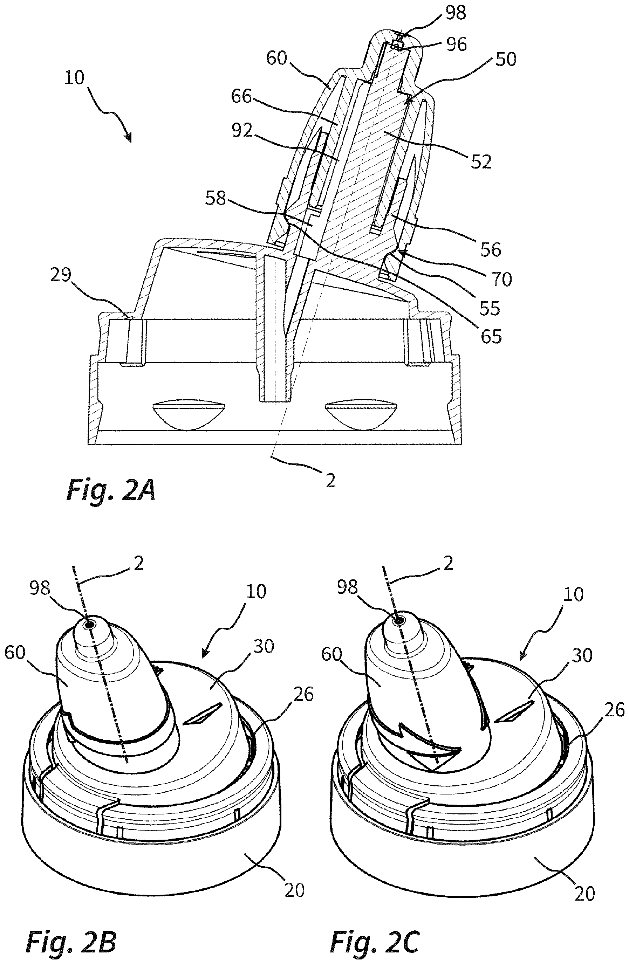

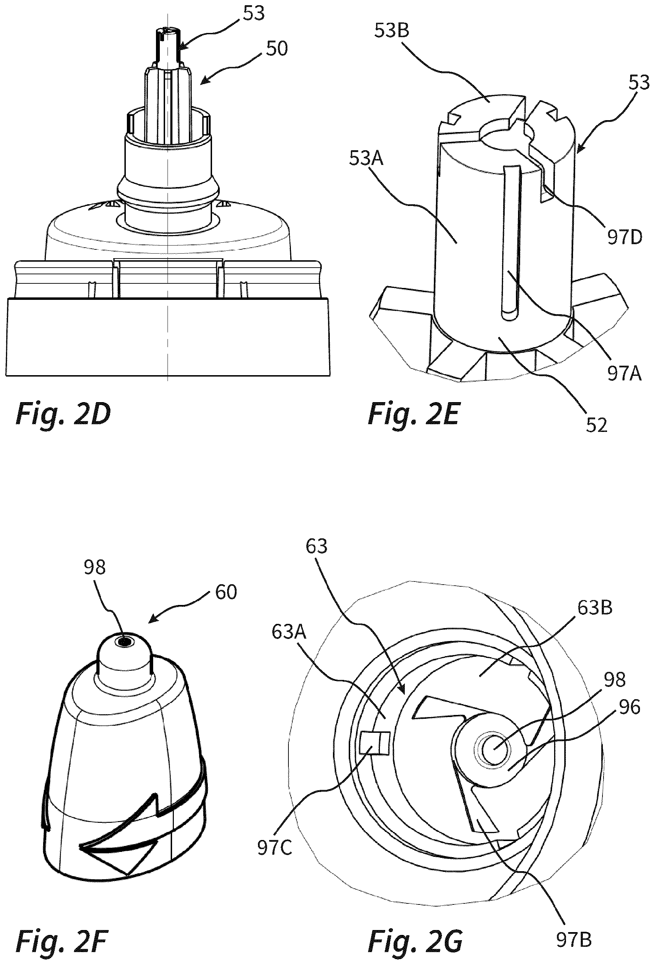

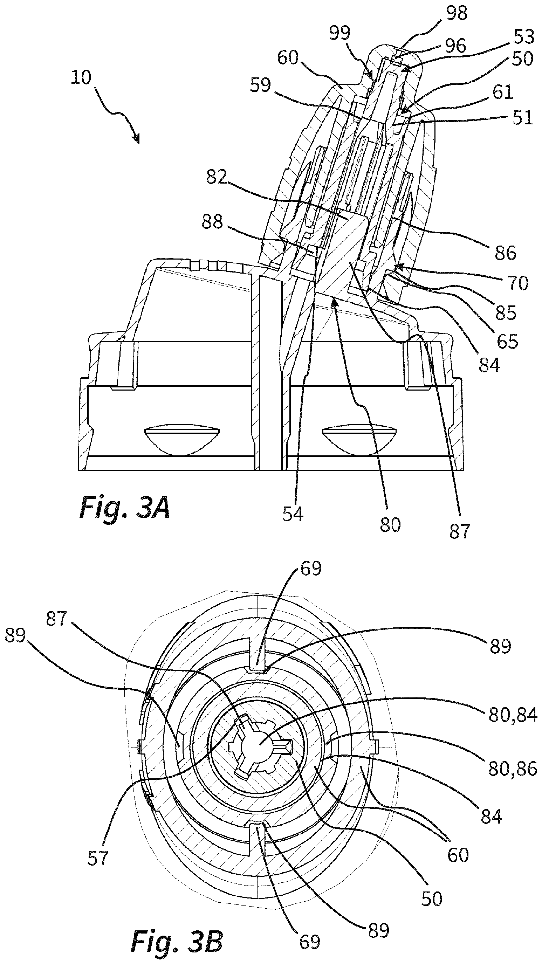

Die Fig. 1A und 1B zeigen eine Gesamtdarstellung eines erfindungsgemäßen Spenders 100 in geschnittener und ungeschnittener Darstellung. Dabei gelten die hierzu nachfolgend erläuterten Zusammenhänge für verschiedene Ausgestaltungen des Austragkopfes 10 dieses Spenders 100. Details zu zwei möglichen Ausgestaltungen des Austragkopfes 10 werden anschließend anhand der Fig. 2A bis 2I und 3A bis 3H erläutert.

Ein erfindungsgemäßer Spender 100 gemäß den vorliegenden Ausführungsbeispielen verfügt über einen Druckspeicher 110, dessen Außenflächen durch einen metallischen Korpus 118 und einen Deckel 120 gebildet werden. Im Deckel 120 ist ein Auslassventil 112 befestigt, welches durch Niederdrücken eines Ventilstutzens 114 gegen die Kraft einer Ventilfeder 116 geöffnet werden kann, so dass die Flüssigkeit in den Austragkopf 10 einströmt.

Der erfindungsgemäße Spender 100 verfügt am Austragkopf 10 über einen schlanken, länglichen Nasalapplikator 12, dessen Haupterstreckungsrichtung gegenüber der Haupterstreckungsrichtung 3 des Druckspeichers 110 angestellt ist. Der Nasalapplikator 12 ist zum Einschieben in ein Nasenloch eines Benutzers vorgesehen. Andere Applikatortypen können hier je nach Anwendungszweck statt des Nasalapplikators 12 vorgesehen sein.

Der Austragkopf 10 ist im Bereich einer Crimp-Verbindung zwischen dem Korpus 118 und dem Deckel 120 auf den Druckspeicher 110 aufgeschnappt, wobei hierfür eine Kopplungseinrichtung an einer Basis 20 des Austragkopfes vorgesehen ist.

Die Basis 20 des Austragkopfes 10 ist mit der überwiegenden Zahl der funktionalen Elemente des Austragkopfes 10 einstückig verbunden.

So ist über eine Kunststoffbrücke 28 eine Betätigungshandhabe 30 mit Betätigungsfläche 32 einstückig an der Basis 20 angeformt. An dieser Betätigungshandhabe 30 ist ein als Hohlrohr 42 ausgestalteter Stößel 40 einstückig angeformt, der in das Auslassventil 112 des Druckspeichers 110 zum Zwecke der Betätigung und zum Zwecke des Einströmens von Flüssigkeit eingeschoben ist.

Darüber hinaus ist auch ein Innenbauteil 50 des Nasalapplikators 12 des Austragkopfes 10 einstückig mit der Betätigungshandhabe 30 ausgebildet, wobei diesbezüglich ein im Weiteren noch erläuterter Unterschied zwischen den Gestaltungen der Fig. 2A bis 2I und der Fig. 3A bis 3H besteht. Das Innenbauteil50 umfasst einen außenliegenden Hülsenabschnitt 56 und einen innenliegenden Stift52.

Ein von diesem Verbundbauteil getrenntes Bauteil des Austragkopfes 10 ist das Außenbauteil 60 des Nasalapplikators 12, an dessen Stirnseite eine Stirnwandung durch eine Austragöffnung 98 durchbrochen ist. Das Außenbauteil 60 ist auf das Innenbauteil 50 aufgeschoben und bei der Gestaltung der Fig. 2A bis 2I formschlüssig am Innenbauteil 50 befestigt, wie im Weiteren noch erläutert wird.

Die Betätigungshandhabe 30 des Spenders 100 ist in Richtung des Pfeils 6 aufgrund der Verformbarkeit der Kunststoffbrücke 28 verschwenkbar, so dass der Nasalapplikator 12 und der Stößel 40 mit verschwenkt werden. Durch das Verschwenken des Stößels 40 wird das Auslassventil 112 geöffnet und Flüssigkeit strömt durch den Innenkanal 90 des Stößels 40 nach oben, gelangt dann in einen Zuströmbereich 58 zwischen dem Zentralstift 52 und dem Hülsenabschnitt 56 des Innenbauteils 50 des Nasalapplikators 12 und wird von dort aus durch einen gemeinsam vom Innenbauteil 50 und dem Außenbauteil 60 definierten Applikatorkanal 92 bis zur Austragöffnung 98 gefördert, von wo die Flüssigkeit ausgetragen werden kann. Zur Abdichtung der Bauteile 50, 60 gegeneinander weist das Außenbauteil eine Hülse 66 auf, deren Außenseite flüssigkeitsdicht an der innenseitigen Dichtfläche des Hülsenabschnitts 56 anliegt.

Das Außenbauteil 60 ist als begrenzt relativbewegliches Außenbauteil 60 ausgebildet.

Die Fig. 2A bis 2I zeigen im Detail eine erste Ausgestaltung eines erfindungsgemäßen Austragkopfes, entsprechend der Fig. 1A und 1B, der zur Bildung eines Spenders 100 auf einem Druckspeicher 110 zu befestigen ist.

Aus Fig. 2B und 2C ist ersichtlich, dass die Basis 20 und die Betätigungshandhabe 30 mit der Betätigungsfläche 32 zwar einstückig miteinander verbunden sind, durch einen insgesamt etwa 300° überspannenden Spalt 26 jedoch ausreichend voneinander getrennt sind, um nach dem Brechen einer Sicherungsbrücke 29 eine Relativbeweglichkeit zu gestatten.

Bei diesem Ausführungsbeispielen weist der Austragkopf 10 die zwei schon beschriebenen Bauteile auf, nämlich - wie in Fig. 2A gut zu erkennen - das erste Bauteil, welches die Basis 20, die Betätigungshandhabe 30 mit Betätigungsfläche 32 sowie einstückig ein Innenbauteil 50 des Applikators bildet, und das zweite Bauteil, das Außenbauteil 60, welches auf das Innenbauteil 50 des Applikators aufgeschoben und hier im Bereich von Spannflächen 55, 65 verrastet ist.

Bei diesem Ausführungsbeispiel ist weiter vorgesehen, dass entsprechend der Fig. 2B und 2C zwischen verschiedenen Austragcharakteristiken umgeschaltet werden kann, nämlich vorliegend zwischen einem Austrag der Flüssigkeit in Form eines Sprühstrahls einerseits und der Austragung in Form eines unzerstäubten Jets andererseits. Das Umschalten der Konfigurationen findet statt, indem das Außenbauteil 60 gegenüber dem Innenbauteil 50 um 180° um die Achse 2 verdreht wird. Eine axiale Verlagerung von Innenbauteil 50 und Außenbauteil 60 findet dabei nicht statt.

Um die Charakteristik des Austrags durch das Verdrehen zu beeinflussen, ist eine spezielle Formgebung eines distalen Innenraums 63 des Außenbauteils 60 und eines Stirnbereichs 53 des Innenbauteils 50 vorgesehen. Die Fig. 2D bis 2G verdeutlichen dies.

Fig. 2E zeigt den Stirnbereich 53 des Innenbauteils 50. Dieser verfügt über eine zunächst kreiszylindrische Grundform, deren Ausmaße bezogen auf den Durchmesser einer Stirnfläche 53B den Ausmaßen des korrespondierenden distalen Innenraums 63 des Außenbauteils 60 entsprechen, so dass der Stirnbereich 53 unter umlaufender und stirnseitiger abdichtender Anlage in den distalen Innenraum 63 eingefügt ist.

Bezugnehmend auf Fig. 2E ist zu erkennen, dass an einer außenseitigen Zylinderfläche 53A des Stirnbereichs 53 axial erstreckende Versorgungsnuten 97A vorgesehen sind, die sich bis zur Stirnfläche 53B erstrecken. Es handelt sich um insgesamt drei Versorgungsnuten 97A im Abstand von 120° zueinander. Um etwa 30° hierzu versetzt sind drei radiale Zuströmnuten 97D in der Stirnfläche 53B des Stirnbereichs 53 des Innenbauteils 50 vorgesehen, die ebenfalls jeweils um 120° voneinander beabstandet sind und sich jeweils bis zur zylindrischen Außenfläche 53A erstrecken.

Korrespondierend zu dieser Gestaltung mit den Nuten 97A, 97D im Stirnbereich 53 des Innenbauteils 50 sind am Außenbauteil in einer innenseitigen umlaufenden Fläche 63A drei um 120° gegeneinander versetzte Nuten 97C vorgesehen, die sich nicht bis zur stirnseitigen Innenfläche 63B erstrecken, sondern kurz davor ihr jeweiliges Ende finden. In der stirnseitigen Innenfläche 63B selbst sind drei tangential ausgerichtete Zuströmnuten 97B vorgesehen, die tangential in eine Wirbelkammer 96 münden und voneinander ebenfalls um 120° beabstandet sind.

Diese Nutenkonfiguration ist vorgesehen, um in Abhängigkeit der Drehstellung des Außenbauteils 60 gegenüber dem Innenbauteil 50 verschiedene Flüssigkeitspfade zu öffnen und zu schließen.

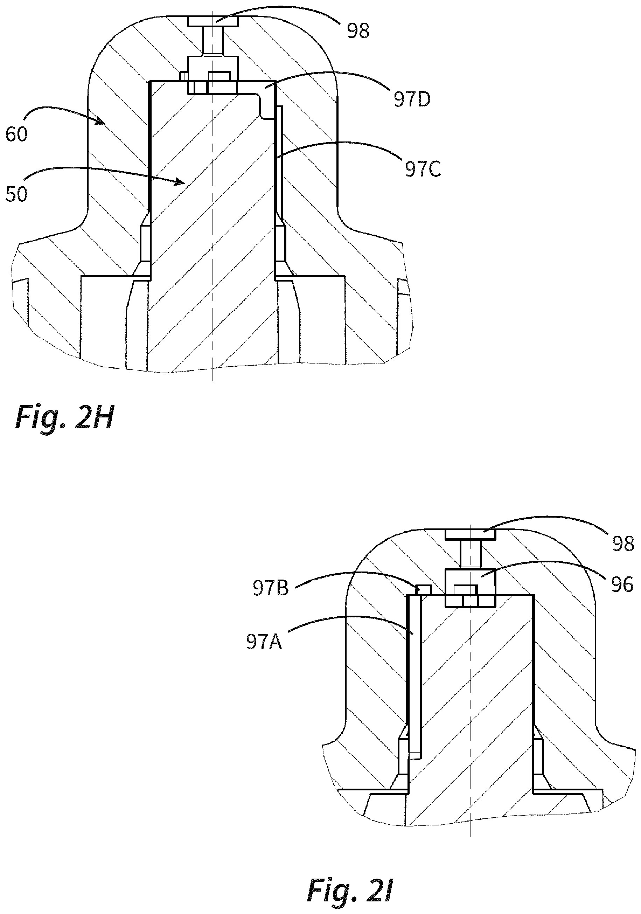

Bezugnehmend auch auf die Fig. 2H und 2I wird das näher erläutert.

Die Fig. 2H zeigt jene Konfiguration, in der das Außenbauteil 60 gegenüber dem Innenbauteil50 derart verdreht ist, dass bestimmungsgemäß ein unzerstäubter Jet ausgetragen werden soll.

In dieser Stellung sind die Versorgungsnuten 97C derart angeordnet, dass Flüssigkeit an ihrem Ende in die radialen Zuströmnuten 97D einströmen kann und so ohne Ausbildung eines Dralls bis zur Austragöffnung 98 gelangen kann. Währenddessen sind in dieser Drehstellung die Tangentialnuten 97B nicht mit Flüssigkeit versorgt, da sie rotativ versetzt zu ihren Versorgungsnuten 97A angeordnet sind.

Wird das Außenbauteil 60 nun um 180° gedreht, so stellt sich die Situation der Fig. 2I ein. In dieser Konfiguration fluchten die Versorgungsnuten 97A mit den Einlässen in die Tangentialnuten 97B. Flüssigkeit kann also in die Tangentialnuten 97B gelangen und in diesen unter Bildung eines Dralls in die Wirbelkammer 96 strömen und als Sprühstrahl durch die Austragöffnung 98 ausgetragen werden. Die Radialnuten 97D, die die Ausbildung des Sprühstrahls stören würden, wenn hier ebenfalls Flüssigkeit einströmen könnte, sind in dieser Drehstellung funktionslos, da sie sich nicht in fluchtender Anordnung zu ihren Versorgungsnuten 97C befinden.

Die beschriebene Nutengestaltung gestattet es somit, sehr funktionssichere und gegeneinander abgedichtete Flüssigkeitspfade sowohl für die Ausbildung eines Sprühstrahls als auch für die Ausbildung eines Jets zur Verfügung zu stellen, wobei die Pfade alleine durch eine Drehbewegung des Außenbauteils gegenüber dem Innenbauteil geöffnet und geschlossen werden können.

In einer dritten Drehstellung zwischen den beiden genannten und um 180° beabstandeten Stellungen sind weder die Versorgungsnuten 97C mit den Zuströmnuten 97D noch die Versorgungsnuten 97A mit den Zuströmnuten 97B in Verbindung, so dass kein Austrag möglich ist.

Damit im Bereich zwischen der Stirnfläche 53B und der stirnseitigen Innenfläche 63B keine Flüssigkeit in die falsche Zuströmnut gelangen kann, ist es wichtig, dass die genannten Flächen möglichst dicht aneinander anliegen. Insbesondere die Tatsache, dass auch eine dichtende Anlage zwischen den zylindrischen Flächen 53A und 53B vorgesehen ist, erschwert diese dichtende Anlage im Stirnbereich. Um sie dennoch zuverlässig zu ermöglichen, sind die bereits genannten Spannflächen 65, 55 vorgesehen, die in Fig. 2A zu erkennen sind. Diese Spannflächen sind gegenüber einer Axialrichtung 2 etwa um 45° geneigt. Das Außenbauteil 60 ist elastisch aufgeweitet, so dass seine Rückstellkraft die einander gegenüberliegenden Spannflächen 65 permanent aufeinander kraftbeaufschlagt. Aufgrund der gegenüberliegenden Spannflächen 55 des Innenbauteils 50 wird infolge dessen das Außenbauteil permanent nach unten kraftbeaufschlagt, so dass die stirnseitige Innenfläche 63B sicher und flächig gegen die Stirnfläche 53B gepresst wird.

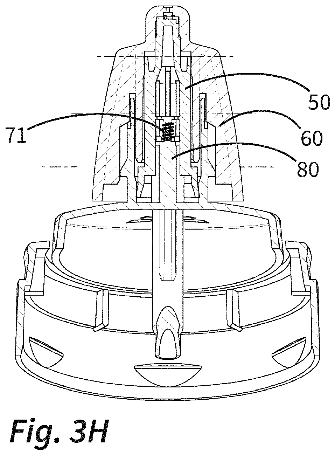

Das Ausführungsbeispiel der Fig. 3A bis 3G mitsamt einer Variante der Fig. 3H weist eine Modifikation auf, die insbesondere dem Zweck dient, die Dichtigkeit im Bereich der Stirnfläche 53B zu gewährleisten sowie durch ein Schaltventil eine Verhinderung eines Austrags besonders sicher zu verhindern.

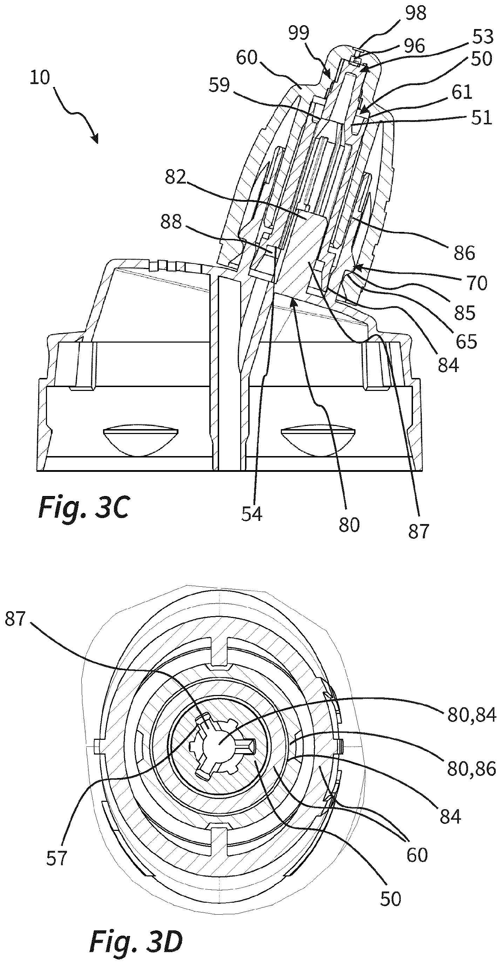

Fig. 3A und 3B zeigen die Stellung von Innenbauteil 50 und Außenbauteil 60 in einer Stellung zur Erzeugung eines Sprühstrahls. Fig. 3C und 3D zeigen die Stellung von Innenbauteil 50 und Außenbauteil 60 in einer Stellung zur Erzeugung eines unzerstäubten Jets.

Bei diesem zweiten Ausführungsbeispiel ist Bezug nehmen auf Fig. 3A das Innenbauteil 50 nicht einstückig mit der Basis 20 und der Betätigungshandhabe 30 verbunden, sondern stattdessen als separates Bauteil 50 ausgebildet, welches axial auf einen Stift 82 aufgeschoben ist, der Teil eines Führungsbauteils 80 ist, welches in diesem konkreten Falle einstückig mit der Betätigungsfläche 32 und der Basis 20 ausgeführt ist. Alternativ könnte jedoch das Führungsbauteil 80 auch separat von den Komponenten 32 und 20 ausgebildet sein und an einer derer lediglich fest angebracht sein.

Ähnlich wie bei der Gestaltung der Fig. 2A bis 2I ist auch bei dieser Gestaltung einer Federeinrichtung 70 vorgesehen, die durch Spannflächen 65, 85 gebildet wird, die in diesem Falle am Führungsbauteil 80 und dem Außenbauteil 60 vorgesehen sind und das Außenbauteil 60 stirnseitig gegen das Innenbauteil 50 drücken.

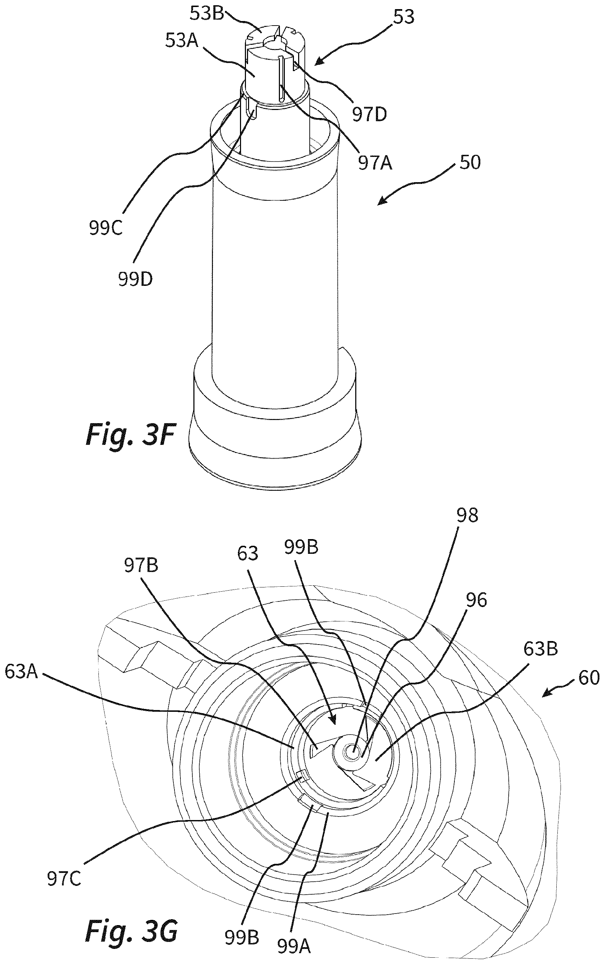

Das Innenbauteil 50, das in Fig. 3F nochmals separat dargestellt ist, weist eine beidseitig offene Hülsenstruktur auf. Mit seinem von der Austragöffnung 98 weg weisenden Ende ist es auf den genannten Stift 82 des Führungsbauteils 80 aufgeschoben. Dabei ist durch Rippen 87 und Ausnehmungen 57 in der in Fig. 3B und 3D verdeutlichten Weise eine Verdrehsicherung geschaffen. Das Innenbauteil 50 ist also drehgesichert, aber grundsätzlich axial verschieblich, am Führungsbauteil 80 angebracht. Das Innenbauteil 50 weist endseitig eine Kolbenfläche 54 auf und liegt mit einer Außenseite dichtend an einer Gleitfläche 84 eines Hülsenabschnitts 86 des Führungsbauteils 80 an. Am gegenüberliegenden Ende des Innenbauteils 50 geht dieses in einen Stirnbereich 53 über, der bezüglich seiner Struktur und insbesondere der Versorgungsnuten 97A und 97D der Struktur entsprechend Fig. 2E entspricht. Um Flüssigkeit hierhin fördern zu können, ist das Innenbauteil auch in diese Richtung offen ausgebildet und weist hierfür eine Durchbrechung 59 auf. Jenseits dieser Durchbrechung ist eine weitere Kolbenfläche 51 vorgesehen, die außenseitig an einer Gleitfläche 61 des Außenbauteils 60 anliegt.

Diese zweite Kolbenfläche 51 bzw. ihr wirksamer Querschnitt ist kleiner als die Kolbenfläche 54 bzw. ihr wirksamer Querschnitt. Dies hat zur Folge, dass unter Druck zugeführte Flüssigkeit das Innenbauteil gegen das Außenbauteil presst. Im Bereich der Stirnfläche 53B und der stirnseitigen Innenfläche 63B wird somit bei anliegendem Druck stets ein dichtes flächiges Anpressen erzielt.

Die Fig. 3B und 3D verdeutlichen weiterhin Raststellungen des Innenbauteils 50 und des Außenbauteils 60 zueinander, wobei im Falle der Gestaltung der Fig. 3A bis 3H diese Raststellungen durch außenseitige Vertiefungen 89 am Hülsenabschnitt 86 des Führungsbauteils 80 sowie nach innen weisende Raststege 69 am Außenbauteil 60 ermöglicht werden. Wenn die Raststege 69 in die Vertiefungen 89 eingerückt sind, bedarf es einer erhöhten Kraft, um das Außenbauteil 60 gegenüber dem Innenbauteil 50 und dem Führungsbauteil 80 zu verdrehen. Werden die Bauteile gegeneinander verdreht, so schnappen die Raststege 69 in die Vertiefungen 89 ein und signalisieren dem Benutzer somit, dass es sich um eine besondere Drehstellung handelt.

Neben den Stellungen der Fig. 3B und 3D, die die Stellung zum Ausbringen eines Sprühstrahls bzw. eines Jets sind, sind zwei weitere Stellungen möglich, die demgegenüber um 90° verdreht sind. In dieser Stellungen ist der Austragkopf gesperrt, wie im Weiteren noch erläutert wird.

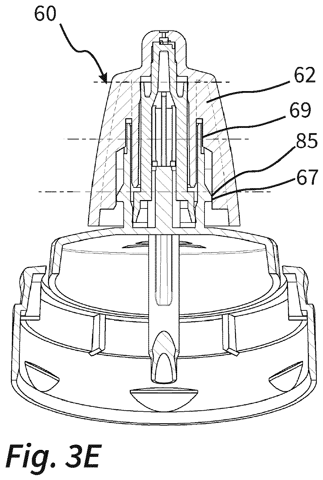

Fig. 3E zeigt einer weitere Schnittdarstellung. Es ist hier zu erkennen, dass das Außenbauteil 60 mit nach innen weisenden radialen Rippen 62 versehen ist. Diese weisen eine Formgebung auf, mit der sie in einem oberen Bereich die genannten Raststege 69 zur Bildung der Raststellungen bilden. Ein unterer Bereich 67 dagegen liegt außenseitig an der Spannfläche 85 an. Wirkung dieses unteren Bereichs 67 ist, dass das Außenbauteil 60 nur in geringem Maße verformbar ist und daher keine Gefahr besteht, dass es sich unter der Wirkung von Flüssigkeitsdruck von dem Innenbauteil 50 und dem Führungsbauteil 80 löst.

Die Fig. 3F und 3G zeigen ähnlich den Figuren 2E und 2G die Zuströmnuten 97B, 97D und die Versorgungsnuten 97A, 97C. Deren Funktionsweise entspricht der bereits erläuterten: Durch Änderung der Drehstellung zwischen dem Innenbauteil 50 und dem Außenbauteil 60 wird die Flüssigkeit entweder entlang eines ersten Flüssigkeitspfades durch die Versorgungsnuten 97A in die Zuströmnuten 97B oder entlang eines zweiten Flüssigkeitspfades durch die Versorgungsnuten 97C in die Zuströmnuten 97D geleitet. Ist keiner dieser Flüssigkeitspfade offen, so kann die Flüssigkeit nicht zur Austragöffnung 98 gelangen.

Bei der Gestaltung der Fig. 3F und 3G ist jedoch zusätzlich ein Schaltventil 99 vorgesehen, welches durch zwei Stufen 99A, 99C im Außenbauteil 60 und Innenbauteil 50 sowie durch darin vorgesehene Unterbrechungen 99B, 99D gebildet wird. In zwei um 180° voneinander beabstandeten Drehstellungen, nämlich genau jenen Drehstellungen für den Austrag als Jet oder Sprühstrahl, sind die Unterbrechungen 99B, 99D derart ausgerichtet, dass Flüssigkeit durch das Schaltventil 99 hindurchfließen kann. In allen anderen Stellungen unterbindet das Schaltventil 99 die Flüssigkeitszufuhr zu den Versorgungsnuten 97A, 97C.

Wie oben bereits beschrieben, ist die kolbenartige Ausgestaltung des Innenbauteils 50 geeignet, dieses bei anliegendem Flüssigkeitsdruck permanent in Richtung der Austragöffnung 98 zu drücken. Hierdurch wird ein besonderes Maß an Dichtigkeit zwischen den aneinander anliegenden Flächen 53B, 63B geschaffen. Die besondere Dichtheit sorgt dafür, dass sowohl die Ausbringung eines Jets als auch eines Sprühstrahls in besonders guter Qualität möglich ist. Flüssigkeit gelangt nur durch die Nuten 97A - 97D zur Austragöffnung 98, nicht aber durch einen ungewollten Spalt zwischen der Stirnfläche 53B und der stirnseitigen Innenfläche 63B.

Das besondere Maß an Dichtigkeit wird darüber hinaus auch im Bereich der Stufen 99A, 99C erzielt, so dass bei geschlossenem Schaltventil 99 selbst eine Betätigung der Betätigungshandhabe 30 und ein Öffnen des Ventils 112 keinen Flüssigkeitsaustrag durch die Austragöffnung 98 zur Folge hat.

Fig. 3H zeigt eine etwas geänderte Variante vom Ausführungsbeispiel der Fig. 3A bis 3G. Hier ist eine zusätzliche Federeinrichtung 71 in Form einer Schraubendruckfeder zwischen dem Führungsbauteil 80 und dem Innenbauteil 50 vorgesehen, die das Innenbauteil 50 permanent stirnseitig an das Außenbauteil 60 anpresst. Bei einer solchen Ausgestaltung kann auf die kolbenartige Wirkungsweise des Innenbauteils 50 verzichtet werden. Vorzugsweise bleibt diese jedoch trotz der Federeinrichtung 71 erhalten.

## Patentansprüche

1. Austragkopf (10) für die nasale Applikation einer pharmazeutischen Flüssigkeit aus einem Druckspeicher (110), welcher über ein Auslassventil (112) mit einem Ventilstutzen (114) verfügt, der gegen eine Federkraft zum Öffnen des Auslassventils (112) kraftbeaufschlagbar ist, mit den folgenden Merkmalen:
a. der Austragkopf (10) verfügt über einen Nasalapplikator (12) zur Einführung in das Nasenloch eines Nutzers, wobei der Nasalapplikator (12) eine länglichem schlanke und zu einem distalen Ende sich verjüngende Formgebung aufweist und wobei am distalen Ende des Nasalapplikators (12) eine Austragöffnung (98) vorgesehen ist, die durch einen Applikatorkanal (92) des Nasalapplikators (12) hindurch mit einem Einlass des Nasalapplikators verbunden ist,
b. der Nasalapplikator (12) verfügt über ein Innenbauteil (50), und
c. der Nasalapplikator (12) verfügt über ein Außenbauteil (60), welches als vom Innenbauteil (50) getrenntes Bauteil ausgebildet ist und das Innenbauteil (50) umgebend an diesem rotativ beweglich angebracht ist,
d. die Austragöffnung (98) ist als Durchbrechung des Außenbauteils (60) vorgesehen, die innenseitig von einer stirnseitigen Innenfläche (63B) umgeben ist, und
e. der stirnseitigen Innenfläche (63B) gegenüberliegend ist eine Stirnfläche (53B) des Innenbauteils (50) vorgesehen, welche flächig an der stirnseitigen Innenfläche (63B) anliegt, und
f. an der stirnseitigen Innenfläche (63B) des Außenbauteils (60) und/oder der Stirnfläche (53B) des Innenbauteils (50) sind mindestens zwei Zuströmkanäle als Zuströmnuten (97B, 97D) ausgebildet, die in Abhängigkeit einer Drehstellung des Außenbauteils (60) zum Innenbauteil (50) mit einem Zuströmbereich (58) stromaufwärts der Zuströmnuten (97B, 97D) verbunden oder hiervon getrennt werden.

2. Austragkopf (10) nach Anspruch 1 mit dem folgenden Merkmal:
a. es sind Zuströmnuten (97B, 97D) zweier Arten vorgesehen, nämlich mindestens eine im Wesentlichen radial ausgerichtete Radialnut (97D) und mindestens eine demgegenüber bezogen auf die Austragöffnung angewinkelte Tangentialnut (97B),
vorzugsweise mit dem folgenden zusätzlichen Merkmal:
b. es ist eine Mehrzahl von Radialnuten (97D) und/oder eine Mehrzahl von Tangentialnuten (97B) vorgesehen.

3. Austragkopf (10) nach Anspruch 1 oder 2 mit dem folgenden Merkmal:
a. es ist mindestens eine Zuströmnut (97B) in der stirnseitigen Innenfläche (63B) des Außenbauteils (60) und mindestens eine Zuströmnut (97D) in der Stirnfläche (53B) des Innenbauteils (50) vorgesehen,
vorzugsweise mit dem folgenden zusätzlichen Merkmal:
b. die mindestens eine Radialnut (97D) ist an der stirnseitigen Innenfläche des Außenbauteils oder der Stirnfläche (53B) des Innenbauteils (50) vorgesehen und die mindestens eine Tangentialnut (97B) ist an der gegenüberliegenden Fläche (63B) vorgesehen.

4. Austragkopf (10) nach einem der vorstehenden Ansprüche mit den folgenden Merkmalen:
a. angrenzend an die stirnseitige Innenfläche (63B) des Außenbauteils (60) weist dieses eine zylindrische Innenfläche (63A) auf, und
b. angrenzend an die Stirnfläche (53B) des Innenbauteils (50) weist dieses eine zylindrische Außenfläche (53A) auf, und
c. die zylindrische Innenfläche (63A) und die zylindrische Außenfläche (53A) liegend dichtend aneinander an, und
d. mindestens eine der beiden Flächen umfassend die zylindrische Innenfläche (63A) und die zylindrische Außenfläche (53A) ist mit einer Versorgungsnut (97A, 97C) versehen, durch die hindurch mindestens eine Zuströmnut (97B, 97D) mit Flüssigkeit aus dem Zuströmbereich (58) versorgbar ist.

5. Austragkopf (10) nach Anspruch 4 mit dem folgenden Merkmal:
a. sowohl in der zylindrischen Innenfläche (63A) als auch in der zylindrischen Außenfläche (53A) ist mindestens eine Versorgungsnut (97A, 97C) zur Versorgung der mindestens einen Zuströmnut (97B, 97D) mit Flüssigkeit aus dem Zuströmbereich (58) versehen.

6. Austragkopf (10) nach Anspruch 4 oder 5 mit mindestens einem der folgenden Merkmale:
a. in der zylindrischen Außenfläche (53A) des Innenbauteils (50) ist mindestens eine Versorgungsnut (97A) vorgesehen, die sich bis zur Stirnfläche (53B) des Innenbauteils erstreckt und/oder
b. in der zylindrischen Innenfläche (63A) des Außenbauteils (60) ist mindestens eine Versorgungsnut (97C) vorgesehen, die sich nicht bis zur stirnseitigen Innenfläche (63B) des Außenbauteils (60) erstreckt.

7. Austragkopf (10) nach einem der vorstehenden Ansprüche mit dem folgenden weiteren Merkmal:
a. im Zuströmbereich (58) stromaufwärts der Zuströmnuten (97B, 97D) ist ein Schaltventil (99) vorgesehen, welches in Abhängigkeit der Drehstellung des Außenbauteils (60) zum Innenbauteil (50) eine Flüssigkeitszufuhr zu den Zuströmnuten (97B, 97D) unterbricht,
vorzugweise mit dem zusätzlichen Merkmal:
b. das Schaltventil ist stromaufwärts von Versorgungsnuten (97A, 97C) zur Versorgung der Zuströmnuten (97B, 97D) vorgesehen.

8. Austragkopf (10) nach einem der vorstehenden Ansprüche mit dem folgenden zusätzlichen Merkmal:
a. das Innenbauteil (50) und das Außenbauteil (60) werden mit einer Federeinrichtung (70) aneinander gepresst,
insbesondere mit dem folgenden zusätzlichen Merkmal:
b. das Außenbauteil (60) und das Innenbauteil (50) weisen schräggestellte Spannflächen (55, 65) auf, die durch elastische Verformung des Innenbauteils (50) oder des Außenbauteils (60) in einer Radialrichtung aneinander angepresst werden und hierdurch mittelbar das Außenbauteil (60) und das Innenbauteil (50) aneinander anpressen.

9. Austragkopf (10) nach Anspruch 8 mit dem folgenden zusätzlichen Merkmal:
a. das Innenbauteil (50) und/oder das Außenbauteil (60) weisen eine von der Kreisform abweichende Formgebung der Spannflächen (55, 65) oder anderer Kontaktflächen auf, so dass bei einer Rotation des Außenbauteils (60) gegenüber dem Innenbauteil (50) mindestens eines der Bauteile (50, 60) eine variierende Verformung zeigt, die insbesondere in zwei Endlagen minimal ist.

10. Austragkopf (10) nach einem der vorstehenden Ansprüche mit den folgenden zusätzlichen Merkmalen:
a. der Austragkopf (10) verfügt über eine Basis (20) und eine daran vorgesehene Kopplungseinrichtung, mittels derer der Austragkopf (10) am Druckspeicher (110) befestigbar ist, und
b. der Austragkopf (10) verfügt über eine verlagerbare Betätigungsfläche (32), die gegenüber der Basis (20) verlagerbar ist, und an der ein als Hohlrohr (42) ausgestalteter Stößel (40) vorgesehen ist, der zum Zwecke der Kraftbeaufschlagung des Ventilstutzens (114) des Druckspeichers (110) gemeinsam mit der Betätigungsfläche (32) verlagerbar ist, und
c. der Nasalapplikator (12) erstreckt sich von der Betätigungsfläche (32) aus nach außen.

11. Austragkopf (10) nach Anspruch 10 mit dem folgenden zusätzlichen Merkmal:
a. das Innenbauteil (50) des Nasalapplikators (12) ist einstückig mit der Betätigungsfläche (32) verbunden.

12. Austragkopf (10) nach Anspruch 10 mit den folgenden zusätzlichen Merkmalen:
a. der Austragkopf weist ein Führungsbauteil (80) auf, und
b. das Innenbauteil (50) ist axial verschieblich und rotativ fixiert am Führungsbauteil (80) angebracht, und
c. das Außenbauteil (60) ist am Führungsbauteil (80) drehbar befestigt,
insbesondere mit dem folgenden zusätzlichen Merkmal:
d. das Führungsbauteil (80) ist einstückig mit der Betätigungsfläche (32) und/oder der Basis (20) ausgebildet ist.

13. Austragkopf (10) nach Anspruch 12 mit dem folgenden zusätzlichen Merkmal:
a. das Innenbauteil (50) und das Führungsbauteil (80) begrenzen gemeinsam eine Drucckammer (88), wobei das Innenbauteil (50) eine zur Druckkammer (88) gewandte Kolbenfläche (54) aufweist, deren Kraftbeaufschlagung durch Flüssigkeitsdruck in der Drucckammer (88) in Richtung der Austragöffnung (98) wirkt.

14. Austragkopf (10) nach einem der vorstehenden Ansprüche mit dem folgenden zusätzlichen Merkmal:
a. es ist eine Federeinrichtung (70) vorgesehen, die zwischen dem Führungsbauteil (80) und dem Außenbauteil (60) wirkt, und/oder es ist eine Federeinrichtung (71) vorgesehen, die zwischen dem Führungsbauteil (80) und dem Innenbauteil (50) wirkt,
insbesondere mit mindestens einem derfolgenden zusätzlichen Merkmale:
b. das Außenbauteil (60) und das Führungsbauteil (80) weisen schräggestellte Spannflächen (85, 65) auf, die durch elastische Verformung des Führungsbauteils (80) oder des Außenbauteils (60) in einer Radialrichtung aneinander angepresst werden und hierdurch mittelbar das Außenbauteil (60) und das Führungsbauteil (80) aneinander anpressen, und/oder
c. das Führungsbauteil (80) und/oder das Außenbauteil (60) weisen eine von der Kreisform abweichende Formgebung der Spannflächen (85, 65) oder anderer Kontaktflächen (69,89) auf, so dass bei einer Rotation des Außenbauteils (60) gegenüber dem Führungsbauteil (80) mindestens eines der Bauteile (80, 60) eine variierende Verformung zeigt, die insbesondere in zwei Endlagen oder in zwei Endlagen und mindestens in einer Zwischenlage minimal ist, und/oder
d. und zwischen dem Führungsbauteil (80) und dem Innenbauteil (50) ist eine Druckfeder (71) vorgesehen, die das Innenbauteil (50) gegen das Außenbauteil (60) drückt.

15. Spender (100) zum Austrag pharmazeutischer Flüssigkeiten mit den folgenden Merkmalen:
a. der Spender (100) verfügt über einen Druckspeicher (110), in welchem pharmazeutische Flüssigkeit unter Druck gelagert ist und der seinerseits über eine Auslassventil (112) verfügt, und
b. das Auslassventil (112) verfügt über einen Ventilstutzen (114), der gegen eine Federkraft zum Öffnen des Ventils kraftbeaufschlagbar ist, und
c. der Spender (100) verfügt über einen Austragkopf (10) zur Ankoppelung an den Druckspeicher (110),
**gekennzeichnet durch** das Merkmal:
d. der Austragkopf (10) ist nach einem der vorstehenden Ansprüche ausgebildet.

## Claims

1. Discharge head (10) for the nasal application of a pharmaceutical liquid from a pressure reservoir (110) which has an outlet valve (112) with a valve connector (114), to which force can be applied counter to a spring force in order to open the outlet valve (112), comprising the following features:
a. the discharge head (10) has a nasal applicator (12) for introduction into the nostril of a user, wherein the nasal applicator (12) has an elongate and slender shaping tapering to a distal end, and wherein a discharge opening (98) is provided at the distal end of the nasal applicator (12), said discharge opening being connected to an inlet of the nasal applicator through an applicator channel (92) of the nasal applicator (12),
b. the nasal applicator (12) has an inner component (50), and
c. the nasal applicator (12) has an outer component (60) which is designed as a component separate from the inner component (50) and is attached in a rotationally movable manner to the inner component (50) surrounding the latter,
d. the discharge opening (98) is provided as an opening in the outer component (60) and is surrounded on the inner side by an end-side inner surface (63B), and
e. opposite the end-side inner surface (63B) an end surface (53B) of the inner component (50) is provided, said end surface lying flat against the end-side inner surface (63B), and
f. at least two inflow channels are designed as inflow grooves (97B, 97D) on the end-side inner surface (63B) of the outer component (60) and/or the end surface (53B) of the inner component (50), said inflow grooves being connected to an inflow region (58) upstream of the inflow grooves (97B, 97D) or being separated therefrom depending on a rotational position of the outer component (60) with respect to the inner component (50).

2. Discharge head (10) according to Claim 1, comprising the following feature:
a. inflow grooves (97B, 97D) of two types are provided, namely at least one substantially radially oriented radial groove (97D) and at least one tangential groove (97B) which is angled in relation thereto with respect to the discharge opening,
preferably comprising the following additional feature:
b. a plurality of radial grooves (97D) and/or a plurality of tangential grooves (97B) are/is provided.

3. Discharge head (10) according to Claim 1 or 2, comprising the following feature:
a. at least one inflow groove (97B) is provided in the end-side inner surface (63B) of the outer component (60) and at least one inflow groove (97D) is provided in the end surface (53B) of the inner component (50),
preferably comprising the following additional feature:
b. the at least one radial groove (97D) is provided on the end-side inner surface of the outer component or the end surface (53B) of the inner component (50), and the at least one tangential groove (97B) is provided on the opposite surface (63B) .

4. Discharge head (10) according to one of the preceding claims, comprising the following features:
a. adjacent to the end-side inner surface (63B) of the outer component (60), the latter has a cylindrical inner surface (63A), and
b. adjacent to the end surface (53B) of the inner component (50), the latter has a cylindrical outer surface (53A), and
c. the cylindrical inner surface (63A) and the cylindrical outer surface (53A) lie in a sealing manner against each other, and
d. at least one of the two surfaces comprising the cylindrical inner surface (63A) and the cylindrical outer surface (53A) is provided with a supply groove (97A, 97C) through which at least one inflow groove (97B, 97D) can be supplied with liquid from the inflow region (58) .

5. Discharge head (10) according to Claim 4, comprising the following feature:
a. at least one supply groove (97A, 97C) for supplying the at least one inflow groove (97B, 97D) with liquid from the inflow region (58) is provided both in the cylindrical inner surface (63A) and in the cylindrical outer surface (53A) .

6. Discharge head (10) according to Claim 4 or 5, comprising at least one of the following features:
a. at least one supply groove (97A) which extends as far as the end surface (53B) of the inner component is provided in the cylindrical outer surface (53A) of the inner component (50), and/or
b. at least one supply groove (97C) which does not extend as far as the end-side inner surface (63B) of the outer component (60) is provided in the cylindrical inner surface (63A) of the outer component (60).

7. Discharge head (10) according to one of the preceding claims, comprising the following further feature:
a. a switching valve (99) is provided in the inflow region (58) upstream of the inflow grooves (97B, 97D), said switching valve interrupting the liquid supply to the inflow grooves (97B, 97D) depending on the rotational position of the outer component (60) with respect to the inner component (50),
preferably with the additional feature:
b. the switching valve is provided upstream of supply grooves (97A, 97C) for supplying the inflow grooves (97B, 97D).

8. Discharge head (10) according to one of the preceding claims, comprising the following additional feature:
a. the inner component (50) and the outer component (60) are pressed against each other with a spring device (70),
in particular comprising the following additional feature:
b. the outer component (60) and the inner component (50) have inclined clamping surfaces (55, 65) which are pressed against each other by elastic deformation of the inner component (50) or of the outer component (60) in a radial direction and thereby indirectly press the outer component (60) and the inner component (50) against each other.

9. Discharge head (10) according to Claim 8, comprising the following additional feature:
a. the inner component (50) and/or the outer component (60) have/has a shaping of the clamping surfaces (55, 65) or of other contact surfaces differing from the circular shape, and therefore, during a rotation of the outer component (60) in relation to the inner component (50), at least one of the components (50, 60) shows a varying deformation which is minimal in particular in two end positions.

10. Discharge head (10) according to one of the preceding claims, comprising the following additional features:
a. the discharge head (10) has a base (20) and a coupling device which is provided on the latter and by means of which the discharge head (10) is fastenable to the pressure reservoir (110), and
b. the discharge head (10) has a shiftable actuating surface (32) which is shiftable in relation to the base (20) and on which a plunger (40) configured as a hollow tube (42) is provided, said plunger being shiftable together with the actuating surface (32) for the purpose of applying force to the valve connector (114) of the pressure reservoir (110), and
c. the nasal applicator (12) extends outwards from the actuating surface (32).

11. Discharge head (10) according to Claim 10, comprising the following additional feature:
a. the inner component (50) of the nasal applicator (12) is connected integrally to the actuating surface (32).

12. Discharge head (10) according to Claim 10, comprising the following additional features:
a. the discharge head has a guide component (80), and
b. the inner component (50) is attached to the guide component (80) in an axially displaceable and rotationally fixed manner, and
c. the outer component (60) is fastened rotatably to the guide component (80),
in particular comprising the following additional feature:
d. the guide component (80) is formed integrally with the actuating surface (32) and/or the base (20) .

13. Discharge head (10) according to Claim 12, comprising the following additional feature:
a. the inner component (50) and the guide component (80) together bound a pressure chamber (88), wherein the inner component (50) has a piston surface (54) which faces the pressure chamber (88), and the application of force of which acts by means of liquid pressure in the pressure chamber (88) in the direction of the discharge opening (98).

14. Discharge head (10) according to one of the preceding claims, comprising the following additional feature:
a. a spring device (70) is provided which acts between the guide component (80) and the outer component (60), and/or a spring device (71) is provided which acts between the guide component (80) and the inner component (50),
in particular comprising at least one of the following additional features:
b. the outer component (60) and the guide component (80) have inclined clamping surfaces (85, 65) which are pressed against each other by elastic deformation of the guide component (80) or of the outer component (60) in a radial direction and thereby indirectly press the outer component (60) and the guide component (80) against each other, and/or
c. the guide component (80) and/or the outer component (60) have/has a shaping of the clamping surfaces (85, 65) or of other contact surfaces (69, 89) differing from the circular shape, and therefore, during a rotation of the outer component (60) in relation to the guide component (80), at least one of the components (80, 60) shows a varying deformation which is minimal in particular in two end positions, or in two end positions and at least in one intermediate position, and/or
d. and a compression spring (71) is provided between the guide component (80) and the inner component (50) and presses the inner component (50) against the outer component (60).

15. Dispenser (100) for discharging pharmaceutical liquids, comprising the following features:
a. the dispenser (100) has a pressure reservoir (110) in which pharmaceutical liquid is stored under pressure and which, for its part, has an outlet valve (112), and
b. the outlet valve (112) has a valve connector (114), to which force can be applied counter to a spring force in order to open the valve, and
c. the dispenser (100) has a discharge head (10) for the coupling to the pressure reservoir (110),
**characterized by** the feature:
d. the discharge head (10) is designed according to one of the preceding claims.

## Revendications

1. Tête de distribution (10) destinée à l'application nasale d'un liquide pharmaceutique provenant d'un réservoir de pression (110) qui dispose d'une soupape de sortie (112) pourvue d'un ajutage de valve (114) qui peut être actionné par une force s'opposant à une force de ressort pour ouvrir la soupape de sortie (112), la tête de distribution présentant les caractéristiques suivantes :
a. la tête de distribution (10) dispose d'un applicateur nasal (12) destiné à être introduit dans la narine d'un utilisateur, l'applicateur nasal (12) ayant une forme allongée et mince se rétrécissant vers une extrémité distale et une ouverture de distribution (98) étant prévue à l'extrémité distale de l'applicateur nasal (12), laquelle ouverture est reliée à une entrée de l'applicateur nasal par le biais d'un conduit (92) de l'applicateur nasal (12),
b. l'applicateur nasal (12) dispose d'un composant intérieur (50), et
c. l'applicateur nasal (12) dispose d'un composant extérieur (60) qui est conçu comme un composant qui est séparé du composant intérieur (50) et qui est fixé de manière rotative au composant intérieur (50) en entourant celui-ci,
d. l'ouverture de distribution (98) est prévue sous la forme d'un passage qui est ménagé dans le composant extérieur (60) et qui est entouré du côté intérieur par une surface intérieure côté frontal (63B), et
e. une surface frontale (53B) du composant intérieur (50) est prévue en face de la surface intérieure côté frontal (63B) et vient en appui de manière sensiblement bidimensionnelle sur la surface intérieure côté frontal (63B), et
f. au moins deux conduits d'entrée se présentant sous la forme de rainures d'entrée d'écoulement (97B, 97D) sont formés sur la surface intérieure côté frontal (63B) du composant extérieur (60) et/ou la surface frontale (53B) du composant intérieur (50) et sont reliés à une zone d'entrée d'écoulement (58) en amont des rainures d'entrée d'écoulement (97B, 97D), ou séparés de celle-ci, en fonction d'une position en rotation du composant extérieur (60) par rapport au composant intérieur (50).

2. Tête de distribution (10) selon la revendication 1 présentant la caractéristique suivante :
a. deux types de rainures d'entrée d'écoulement (97B, 97D) sont prévus, à savoir au moins une rainure radiale (97D) orientée sensiblement radialement et au moins une rainure tangentielle (97B) coudée par rapport à l'ouverture de distribution,
de préférence présentant la caractéristique supplémentaire suivante :
b. une pluralité de rainures radiales (97D) et/ou une pluralité de rainures tangentielles (97B) sont prévues.

3. Tête de distribution (10) selon la revendication 1 ou 2 présentant la caractéristique suivante :
a. au moins une rainure d'entrée d'écoulement (97B) est prévue dans la surface intérieure côté frontal (63B) du composant extérieur (60) et au moins une rainure d'entrée d'écoulement (97D) est prévue dans la surface frontale (53B) du composant intérieur (50),
de préférence présentant la caractéristique supplémentaire suivante :
b. l'au moins une rainure radiale (97D) est prévue sur la surface intérieure côté frontal du composant extérieur ou sur la surface frontale (53B) du composant intérieur (50) et l'au moins une rainure tangentielle (97B) est prévue sur la surface opposée (63B).

4. Tête de distribution (10) selon l'une des revendications précédentes présentant les caractéristiques suivantes :
a. le composant extérieur (60) comporte une surface intérieure cylindrique (63A) adjacente à la surface intérieure côté frontal (63B) de celui-ci, et
b. le composant intérieur (50) comporte une surface extérieure cylindrique (53A) adjacente à la surface frontale (53B) de celui-ci, et
c. la surface intérieure cylindrique (63A) et la surface extérieure cylindrique (53A) viennent en appui l'une sur l'autre de manière étanche, et
d. au moins une des deux surfaces comprenant la surface intérieure cylindrique (63A) et la surface extérieure cylindrique (53A) est pourvue d'une rainure d'alimentation (97A, 97C) permettant d'alimenter au moins une rainure d'entrée d'écoulement (97B, 97D) en liquide provenant de la zone d'entrée d'écoulement (58) .

5. Tête de distribution (10) selon la revendication 4 présentant la caractéristique suivante :
a. la surface intérieure cylindrique (63A) et la surface extérieure cylindrique (53A) comportent toutes deux au moins une rainure d'alimentation (97A, 97C) destinée à alimenter l'au moins une rainure d'entrée d'écoulement (97B, 97D) en liquide provenant de la zone d'entrée d'écoulement (58).

6. Tête de distribution (10) selon la revendication 4 ou 5 présentant l'une au moins des caractéristiques suivantes :
a. la surface extérieure cylindrique (53A) du composant intérieur (50) comporte au moins une rainure d'alimentation (97A) qui s'étend jusqu'à la surface frontale (53B) du composant intérieur et/ou
b. la surface intérieure cylindrique (63A) du composant extérieur (60) comporte au moins une rainure d'alimentation (97C) qui ne s'étend pas jusqu'à la surface intérieure côté frontal (63B) du composant extérieur (60).

7. Tête de distribution (10) selon l'une des revendications précédentes présentant la caractéristique supplémentaire suivante :
a. la zone d'entrée d'écoulement (58) comporte en amont des rainures d'entrée d'écoulement (97B, 97D) une soupape de commutation (99) qui interrompt l'alimentation en liquide des rainures d'entrée d'écoulement (97B, 97D) en fonction de la position en rotation du composant extérieur (60) par rapport au composant intérieur (50),
de préférence présentant la caractéristique supplémentaire :
b. la soupape de commutation est prévue en amont des rainures d'alimentation (97A, 97C) destinées à alimenter les rainures d'entrée d'écoulement (97B, 97D) .

8. Tête de distribution (10) selon l'une des revendications précédentes présentant la caractéristique supplémentaire suivante :
a. le composant intérieur (50) et le composant extérieur (60) sont pressés l'un sur l'autre avec un dispositif à ressort (70),
en particulier présentant la caractéristique supplémentaire suivante :
b. le composant extérieur (60) et le composant intérieur (50) comportent des surfaces de serrage inclinées (55, 65) qui sont pressées l'une sur l'autre dans une direction radiale par déformation élastique du composant intérieur (50) ou du composant extérieur (60) et qui pressent ainsi indirectement le composant extérieur (60) et le composant intérieur (50) l'un sur l'autre.

9. Tête de distribution (10) selon la revendication 8 présentant la caractéristique supplémentaire suivante :
a. le composant intérieur (50) et/ou le composant extérieur (60) ont une forme, s'écartant de la forme circulaire, des surfaces de serrage (55, 65) ou d'autres surfaces de contact, de sorte que, lorsque le composant extérieur (60) tourne par rapport au composant intérieur (50), l'un au moins des composants (50, 60) présente une déformation variable qui est minime notamment dans deux positions extrêmes.

10. Tête de distribution (10) selon l'une des revendications précédentes présentant les caractéristiques supplémentaires suivantes :
a. la tête de distribution (10) dispose d'une base (20) et d'un dispositif d'accouplement, prévu sur celle-ci, qui permettent de fixer la tête de distribution (10) au réservoir sous pression (110), et
b. la tête de distribution (10) dispose d'une surface d'actionnement déplaçable (32) qui peut être déplacée par rapport à la base (20) et sur laquelle est prévu un poussoir (40) qui est conçu comme un tube creux (42) et qui peut être déplacé conjointement avec la surface d'actionnement (32) pour appliquer une force sur l'ajutage de soupape (114) du réservoir sous pression (110), et
c. l'applicateur nasal (12) s'étend vers l'extérieur depuis la surface d'actionnement (32).

11. Tête de distribution (10) selon la revendication 10 présentant la caractéristique supplémentaire suivante :
a. le composant intérieur (50) de l'applicateur nasal (12) est relié d'une seule pièce à la surface d'actionnement (32).

12. Tête de distribution (10) selon la revendication 10 présentant les caractéristiques supplémentaires suivantes :
a. la tête de distribution comporte un composant de guidage (80), et
b. le composant intérieur (50) est fixé au composant de guidage (80) de manière déplaçable axialement et fixe en rotation, et
c. le composant extérieur (60) est fixé de manière rotative au composant de guidage (80),
en particulier présentant la caractéristique supplémentaire suivante :
d. le composant de guidage (80) est formé d'une seule pièce avec la surface d'actionnement (32) et/ou la base (20) .

13. Tête de distribution (10) selon la revendication 12 présentant la caractéristique supplémentaire suivante :
a. le composant intérieur (50) et le composant de guidage (80) délimitent conjointement une chambre sous pression (88), le composant intérieur (50) comportant une surface de piston (54) qui est dirigée vers la chambre sous pression (88) et qui est soumise à une force qui agit en direction de l'ouverture de distribution (98) par le biais de la pression de fluide dans la chambre sous pression (88).

14. Tête de distribution (10) selon l'une des revendications précédentes présentant la caractéristique supplémentaire suivante :
a. un dispositif à ressort (70) est prévu qui agit entre le composant de guidage (80) et le composant extérieur (60) et/ou un dispositif à ressort (71) est prévu qui agit entre le composant de guidage (80) et le composant intérieur (50),
en particulier présentant au moins une des caractéristiques supplémentaires suivantes :
b. le composant extérieur (60) et le composant de guidage (80) comportent des surfaces de serrage inclinées (85, 65) qui sont pressées l'une sur l'autre dans une direction radiale par déformation élastique du composant de guidage (80) ou du composant extérieur (60) et qui pressent ainsi indirectement le composant extérieur (60) et le composant de guidage (80) l'un sur l'autre, et/ou
c. le composant de guidage (80) et/ou le composant extérieur (60) ont une forme, s'écartant de la forme circulaire, des surfaces de serrage (85, 65) ou d'autres surfaces de contact (69, 89) de sorte que, lorsque le composant extérieur (60) tourne par rapport au composant de guidage (80), l'un au moins des composants (80, 60) présente une déformation variable qui est minime notamment dans deux positions extrêmes, ou dans deux positions extrêmes et au moins dans une position intermédiaire, et/ou
d. et un ressort de compression (71) est prévu entre le composant de guidage (80) et le composant intérieur (50) et presse le composant intérieur (50) contre le composant extérieur (60).

15. Distributeur (100) destiné la distribution de liquides pharmaceutiques, le distributeur présentant les caractéristiques suivantes :
a. le distributeur (100) dispose d'un réservoir sous pression (110) dans lequel un liquide pharmaceutique est stocké sous pression et qui dispose à son tour d'une soupape de sortie (112), et
b. la soupape de sortie (112) dispose d'un ajutage de soupape (114) qui peut être soumis à une force, s'opposant à une force de ressort, pour ouvrir la soupape, et
c. le distributeur (100) comporte une tête de distribution (10) destinée à s'accoupler au réservoir sous pression (110),
**caractérisé par** la caractéristique :
d. la tête de distribution (10) est conçue selon l'une des revendications précédentes.
